# EUROPEAN PATENT APPLICATION

(11) **EP 1 245 209 A2**
(43) Date of publication of application: **02.10.2002**
(21) Application number: 02007506.5
(22) Date of filing: 02.04.2002
(51) Int. Cl.: A61F 13/476, A61F 13/505

(54) **Absorbent article with removable wings**

(30) Priority: 30.03.2001 US 823144
(71) Applicant: McNEIL-PPC, INC., Skillman, NJ 08858 (US)
(72) Inventor: Garrod, Christopher T., Middletown, DE 19709 (US); Haarer, Jutta S., Princeton, NJ 08540 (US); Moscherosch, Michael H., Doylestown, PA 19801 (US)
(74) Representative: Groening, Hans Wilhelm, Dipl.-Ing.

(57) **Abstract**

An absorbent article with a silhouette having a first portion having a maximum width, a second portion having a maximum width, wherein the second portion being in opposite relation to the first portion and the maximum width of the first portion is greater than the maximum width of the second portion, and (iii) a first longitudinally extending edge and a second longitudinally extending edge being in opposed relationship between the first portion to the second portion; a layered portion including an absorbent core and a backsheet; at least one wing laterally extending from the first longitudinally extending edge or the second longitudinally extending edge; and means for removing the wing from the longitudinally extending edge.

Alternatively, an attachment wing for securing an absorbent article to a garment with an upper surface including an absorbent article attachment for securing the attachment wing to the absorbent article and a lower surface comprising a garment attachment means securing the attachment wing to the garment. Additionally, a kit is provided having an absorbent article and at least one attachment wing for securing the absorbent article to a garment.

## Description

### Field of the Invention

The invention relates to an absorbent article for use with garments that includes at least one removable wing extending laterally outward from a longitudinal edge of the absorbent article.

### Background of the Invention

Recently, the popularity of thong garments have increased, giving rise to the development of absorbent articles specifically for use with such garments. However, thong garments present unique requirements for an absorbent article. As used herein, the term thong garment includes, but is not limited to, thong underwear, thong swimming suit bottom, G-strings, Rio cut underwear, Rio-cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

U.S. Patent No. 5,713,886 to Sturino discloses a sanitary napkin or pantiliner for use with thong garments. The disclosed article includes an absorbent core with first and second end portions each having opposed sides that extend longitudinally from the ends. The opposed sides of the second portion flare continuously from the sides of the first portion so that the second portion has, at all points along its length, a transverse width greater than that of the first portion. The disclosed pantiliner has wings located both on the first end portion and the second end portion.

U.S. Patent No. Des. 394,503 and UK Design Registration No. 2076491 both to Perrini relate to designs for thong pantiliners having overall shapes similar to a light bulb with side wings extending from the narrow portions of the pantiliners. In each design, the side wings are located on the narrow portion of the pantiliner adjacent to where the narrow portion joins the wider portion of the pantiliner.

Similarly, UK Design Registration No. 2087071 also to Perrini discloses a thong pantiliner having a more gradual change in width along its length, also with side wings. In this case, side wings are located roughly in the middle of the pantiliner.

International Publication No. WO 00/72790 A1 to SCA Hygiene Products AB discloses an absorbent article wherein the fastening tabs, e.g., wings or flaps, are formed from separate pieces of material. The fastening tabs and the edge portion of the article were described as being attached to each other within the coinciding edge portion. In particular, to hold the absorbent article in place in the thong garment, it was posited that it was important for the fastening tabs to be secured either to each or the thong garment by providing the fastening tabs with some form of fastening arrangement, for example, adhesive, hook and loop, snap fasteners, and friction-type fasteners. The application describes a desire to have no direct connection between the cover or the absorption body of the article and the fastening tabs to form a leakage barrier.

The above pantiliners and absorbent articles do not facilitate increased discretion for wearers. In particular, a wearer may not wish to use wings on certain occasions because of their visibility. However, removing wings from the above pantiliners and absorbent articles is difficult and not practicable. Thus, there is a need for an absorbent article for thong garments having removable wings.

U.S Patent No. 5,683,373 to Darby discloses a sanitary napkin for use with thong garments. However, because the above sanitary napkin does not include wings, the sanitary napkin during use is not as secure as may sometimes be required. Therefore, there is a need to attach wings to such a napkin or pantyliner should a wearer desire more secure attachment.

Applicants have surprisingly discovered a highly useful absorbent article for garments and thong garments having at least one removable wing. Such an absorbent article provides a wearer with at least one wing attached to the absorbent article or the wearer may optionally detach some or all the wings. In another embodiment of the present invention, wings may be added by the wearer to the absorbent article to provide additional protection against movement of the pantiliner. Thus, the present invention allows a wearer to individually decide whether to use from zero to three or more wings to secure the absorbent article of the present invention to thong garments.

### Summary of the Invention

The present invention is directed to an absorbent article with a silhouette having a first portion, a second portion, wherein the second portion being in opposite relation to the first portion, and a first longitudinally extending edge and a second longitudinally extending edge being in opposed relationship between the first portion to the second portion; a layered portion including an absorbent core and a backsheet; at least one wing laterally extending from the first longitudinally extending edge or the second longitudinally extending edge; and means for removing the wing from the longitudinally extending edge.

The present invention is also directed to an absorbent article with a silhouette having a first portion having a maximum width, a second portion having a maximum width, wherein the second portion being in opposite relation to the first portion and the maximum width of the first portion is greater than the maximum width of the second portion, and a first longitudinally extending edge and a second longitudinally extending edge being in opposed relationship between the first portion to the second portion; a layered portion including an absorbent core and a backsheet; at least one wing laterally extending from the first longitudinally extending edge or the second longitudinally extending edge; and means for removing the wing from the longitudinally extending edge.

Alternatively, an attachment wing for securing an absorbent article to a garment with an upper surface including an absorbent article attachment for securing the attachment wing to the absorbent article and a lower surface comprising a garment attachment means securing the attachment wing to the garment.

Additionally, a kit is provided having an absorbent article and at least one attachment wing for securing the absorbent article to a garment.

### Brief Description of the Drawings

Figures 1-3 are plan views of absorbent articles according to the present invention having various wings;
Figure 4 is a plan view of a absorbent article according to the present invention having staggered, or non-symmetrical wings;
Figure 5 is a plan view of a absorbent article according to the present invention having wings extending continuously around the posterior end of the pantiliner;
Figure 6 is a plan view of a absorbent article according to the present invention in which the wings are of an irregular shape;
Figure 7 is a plan view of a absorbent according to the present invention in which the midsection is biconcave in shape;
Figure 8 is a plan view of a absorbent according to the present invention in which the first portion is round and the midsection is of a uniform width;
Figure 9 is a plan view of a absorbent article of the present invention with wings showing perforation lines; and

### Detailed Description of the Invention

In one embodiment of the present invention, the absorbent article has a silhouette having a first portion, a second portion, wherein the second portion being in opposite relation to the first portion, and a first longitudinally extending edge and a second longitudinally extending edge being in opposed relationship between the first portion to the second portion; a layered portion including an absorbent core and a backsheet; at least one wing laterally extending from the first longitudinally extending edge or the second longitudinally extending edge; and means for removing the wing from the longitudinally extending edge.

In an alternate embodiment of the present invention, the absorbent article has a silhouette having a first portion having a maximum width and a second portion having a maximum width; wherein the second portion being in opposite relation to the first portion and the maximum width of the first portion is greater than the maximum width of the second portion, and a first longitudinally extending edge and a second longitudinally extending edge being in opposed relationship between the first portion to the second portion. The absorbent article of this embodiment of the present invention also has a layered portion with an absorbent core and a garment-facing backsheet, at least one wing laterally extending from the first longitudinally extending edge or the second longitudinally extending edge, and a means for removing the wing from the longitudinally extending edge.

The absorbent core or layer of the present invention may contain any known absorbent materials including, but not limited to, absorbent fibers, such as cellulose fibers, e.g., wood pulp, regenerated cellulose fibers, and cotton fibers, rayon fibers, superabsorbent polymers, e.g., fibers or particles, other naturally occurring absorbent materials, e.g., peat moss, and other synthetic absorbent materials, such as foams and the like. The absorbent layer may also include one or more of the following: thermoplastic binder fibers, latex binder, perfumes, or odor-controlling compounds. The absorbent layer may be compressed or uncompressed, embossed, or calendered.

The backsheet of the present invention is a body fluid impervious material, typically referred to as a "barrier," at least substantially impermeable to liquids, and its exterior forms the garment-facing surface of the absorbent article. The backsheet may be any thin, flexible, body fluid impermeable material, such as a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene. The thickness of the backsheet when formed from a polymeric film typically is about 0.001 to about 0.002 inch (about 0.00254 to about 0.00508 cm).

Optionally, the backsheet may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. The disclosures of these patents are incorporated herein by reference in their entirety. As disclosed in the above documents, wings are generally speaking flexible and configured to be folded over the edges of the underwear so that the wings are disposed between the edges of the underwear.

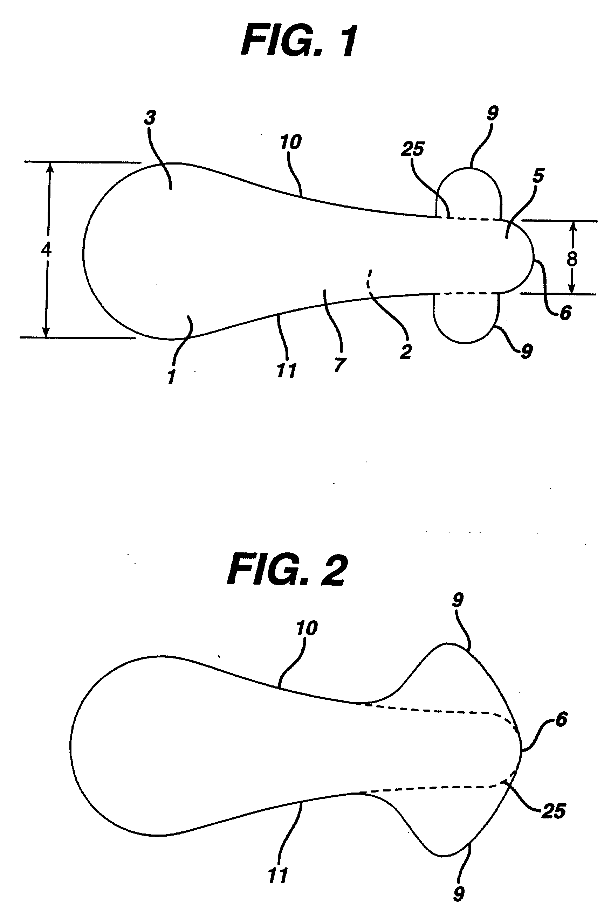

The shape of the wings may be varied. The wings may be rounded, rectangular, square, curvilinear, etc. Examples of wings with different shapes are shown in Figures 1-8. The wings may be regular or irregular, symmetric or asymmetric in shape.

In Figures 1-8, perforation line 25 is depicted as the method of attachment/detachment for removable wings 9, 9a and 9b. However, removable wings of the present invention may be attached to longitudinal edges 10 or 11 of the absorbent article by adhesive, snap, button, hook and loop, staple, stitched, emboss, crimp, friction, and perforation.

As used herein, the term perforation is intended to mean a line of cuts, scores, embossing, and the like. The choice of perforating methods is dependent on the materials and amount of cut required. Commonly used methods include knife cutting, ultrasonic cutting, embossing, and sealing. A partially cutting knife will produce clean cuts through materials with parts of the perforation line not cut. For a sealing or embossing tool, the material would be crushed or fractured along the perforation line to form a stress concentrated area that tears easily away. This would be through the whole construction or through the non-paper release strip.

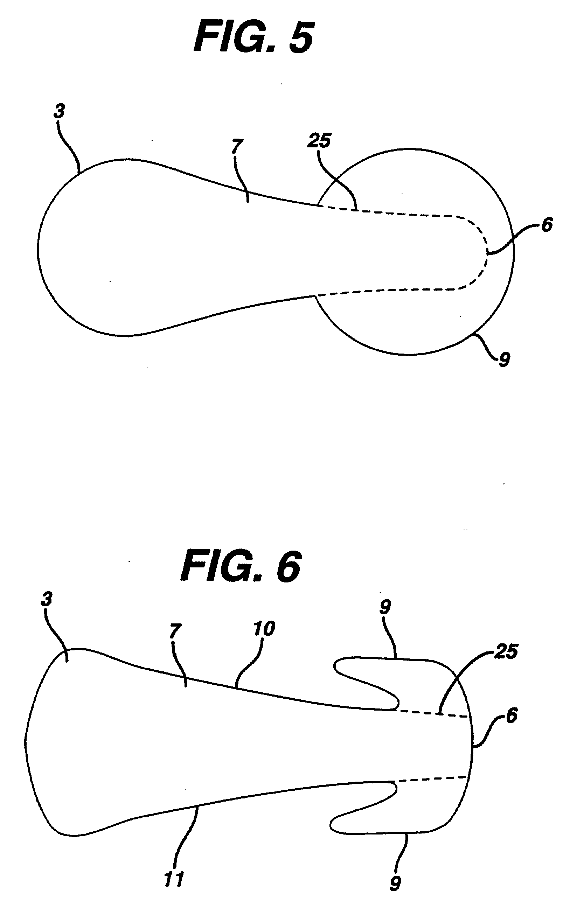

Perforation line 25 may follow a longitudinal edge of the second portion to the second portion end. Alternatively, a single perforation line 25 may permit removal of more than one removable wing 9 as depicted in Figure 5. The actual perforation may be completely through the pantiliner material or almost all the way through, whichever makes for an easy tear. If the perforation is to be made completely through the pantiliner, then it is important that the cuts be made completely through the absorbent core and backsheet layers. The perforation may be done when the product is complete, with or without the paper release strip. It is not necessary to perforate the release paper, if present although the release paper may also be perforated. The perforations, however, must not compromise the strength of the wing when securing the absorbent article to a thong garment or other garment. Additionally, the perforations must be close enough together to ensure easy and clean removal of the wing. Women may prefer to leave the wings attached to the absorbent article, using the wings to further secure the absorbent article to a thong garment. At other time, women may find the extra attachment unnecessary and will remove the wings, attaching the absorbent article to a thong garment without wings. The removable wings allow women a choice of whether to utilize the extra attachment features of the absorbent article.

The design and placement of the removable wings is not critical to the invention. For example, the wings of the present invention may be an extension of the backsheet. If desired, the wings may optionally be provided with a thin layer of absorbent material. Theoretically, only a relatively small amount of discharged fluid should reach the wings, therefore, only a relatively small amount of absorbent material is needed. Such absorbent material may be the same as or different from the absorbent material used to make the absorbent core. For example, the absorbent core may simply extend into the wings. Any absorbent material present in the wings should be relatively flexible. Thus, the wings would be a laminated of the backsheet and the absorbent core.

Alternatively, if a cover is included on the absorbent article, the wings may be a laminate of extensions of the cover and backsheet, or even a laminate of extensions of the cover, absorbent core and backsheet. On the other hand, the wings need not be unitary with the absorbent article, and may be separate elements that are affixed to the absorbent article, i.e., "cut and paste" type wings.

While wings can be made from the same material as the absorbent article, other materials, such as elastic nonwoven materials or films may also be used. It is preferred in any case that the wings have a liquid impervious barrier sheet to prevent exudates that reach the wings from soiling the edges of the wearer's underwear.

The location of wings can be at any location along a longitudinal edge of the absorbent article of the present invention. In a preferred embodiment of the present invention, at least one removable wing is attached to the finished absorbent article, e.g., the wing is put in place during the manufacturing process. In another embodiment of the present invention, at least one wing is provided to the wearer for placement on the absorbent article wherever the wearer desires. In an additional embodiment of the present invention, removable wings 9 shown in Figure 5 are connected to each other and extend continuously around the second portion end 6 of the absorbent article. In this embodiment of the invention, the wings may be an extension of a laminate of the cover and the backsheet, as described above.

The overall dimensions of the absorbent article of the present invention are preferably as follows. The length is preferably in the range of about 5 to about 8 inches (about 12 to about 20 cm). The maximum width of the anterior portion is preferably in the range of about 2 to about 3 inches (about 5 to about 8 cm). The thickness of the absorbent article is preferably in the range of about 0.04 to about 0.2 inches (about 0.1 to about 0.5 cm).

Although not required, the absorbent article of the present invention may include a cover overlaying the absorbent core. The exterior of the cover would then form the body-facing side of the absorbent article. The cover may be formed from any fluid pervious material that is comfortable against the skin and permits fluid to penetrate to the absorbent core, which retains the fluid. The cover should retain little or no fluid to provide a relatively dry surface next the skin when in use. A variety of cover materials are known in the art, and any of these may be used. For instance, the cover may be a fibrous non-woven fabric made of fibers or filaments of polymers, such as polyethylene, polypropylene, polyester, or cellulose, and combinations thereof. Alternatively, the cover may be formed from an apertured polymeric film. The thickness of the cover may vary from about 0.001 to about 0.062 inch (about 0.0025 to about 0.016 cm), depending on the material chosen.

Generally, the optional cover is a single sheet of material having a width sufficient to form the body-facing surface of the absorbent article. Preferably, the cover is longer and wider than the absorbent core.

Optionally, the absorbent article of the present invention may include a transfer layer. If included in the absorbent article, the transfer layer may be made of any known material that will take up fluid and then distribute and release it to an adjacent absorbent core or layer for storage. Preferred transfer layers have a relatively open structure that allows for movement of fluid within the layer. Suitable materials for such transfer layers include fibrous webs, resilient foams, and the like.

The transfer layer is able to accept fluid and allow passage of the fluid through its mass to be absorbed by an adjacent absorbent core. The mass of materials making up the transfer layer may be absorbent, although the materials themselves may not absorbent. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Preferred transfer layer fibrous webs are made of resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials.

As is customary in the art, a paper release strip, which has been coated on one side, is applied to protect adhesive that may be applied to the garment-facing side of the backsheet. The coating on the paper release strip, which may be silicone, reduces the adherency to the adhesive of the coated side of the release strip. The release strip can be formed from any suitable sheet-like material which, when coated, adheres with sufficient tenacity to the adhesive to remain in place prior to use but which can be readily removed when the absorbent article is to be used.

The adhesive applied to the garment facing side of the absorbent article may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include rapid setting thermoplastic "hot melt," rubber adhesives, two-sided adhesive tape, and the like.

Turning to the Figures, Figure 1 depicts an absorbent article according to the present invention. The overall shape of the absorbent article is such that it will fit comfortably within the confines of a thong garment. Referring to Figure 1, the absorbent article includes midsection 7 between a relatively wide first portion 3 with a narrower second portion 5. In particular, the maximum width 4 of the first portion 3 of the pantiliner is greater than the maximum width of the second portion 8 of the second portion end 6 of the absorbent article. First portion 3 is connected to second portion 5 by a pair of opposed longitudinal edges 10 and 11. Figure 1 also depicts absorbent core 1 of the absorbent article on the body-facing surface of the absorbent article and backsheet 2 on the garment-facing side of the absorbent core.

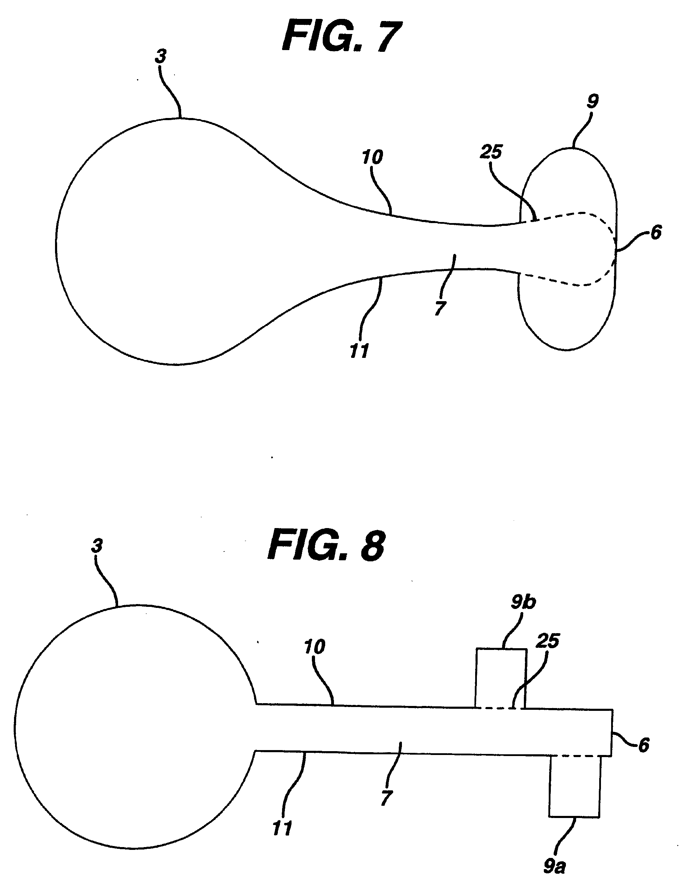

The precise shapes of the first portion and the second portion may vary as desired, so long as the maximum width of the first portion is greater than the maximum width of the second portion. For example, the first portion may have the overall shape of a bulb, as shown in Figures 1-5. Alternatively, the first portion may more triangular as shown in.Figure 6, or may be round as shown in Figure 8. The midsection may be tapered and narrow at a substantially continuous rate along its length, as shown in Figures 1-6. The midsection may be biconcave in shape, as shown in Figure 7. The midsection may also be of a narrow, uniform width like a stem, as shown in Figure 8.

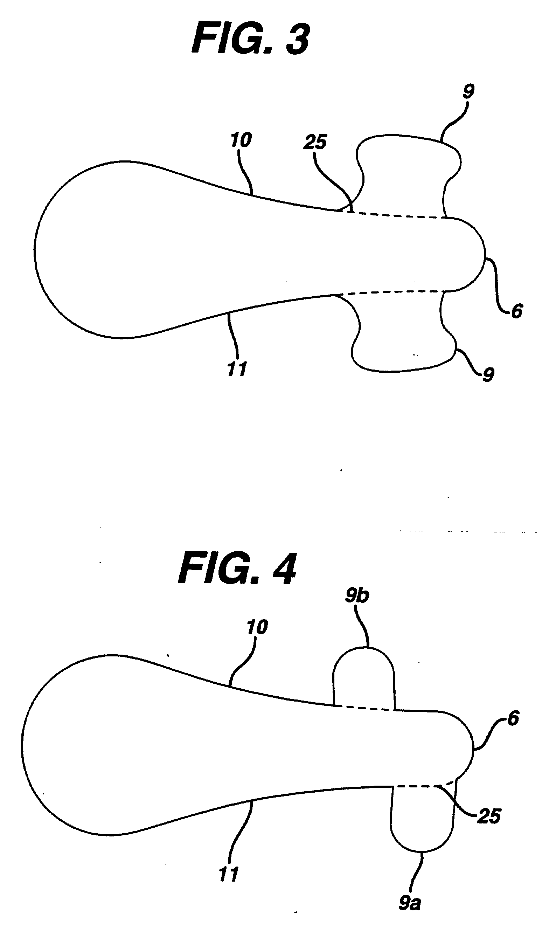

Referring again to Figure 1, removable wings 9 extend laterally from longitudinal edge 10 and longitudinal edge 11 of the absorbent article. The number of removable wings 9 can vary from one to three or more. There is no requirement that removable wings 9 be in opposed symmetry along longitudinal edges 10 and 11. See, for example, removable wings 9a and 9b of Figure 4. Such a non-symmetrical arrangement for removable wings 9a and 9b has the dual advantages of minimizing the thickness of material between the buttocks, as well as fixing a longer portion of the absorbent article more securely to the thong garment.

The Figures show absorbent articles according to the present invention with at least one removable wing 9 or 9a that is either near or adjacent to the second portion end 6 or includes second portion end 6 of the absorbent article. In use, removable wings 9, 9a and 9b are folded over the edge of the thong garment and, depending on their size and shape, may overlap, partially or completely, with one another in folded position.

Figures 4 and 8 show absorbent articles according to the present invention in which the removable wings 9 are staggered. One of the removable wings, 9a, is near to the second portion end 6 of the absorbent article. Removable wing 9b is positioned more remote from the second portion end 6. In use, removable wings 9a and 9b are folded over the edge of the thong garment, but do not overlap one another.

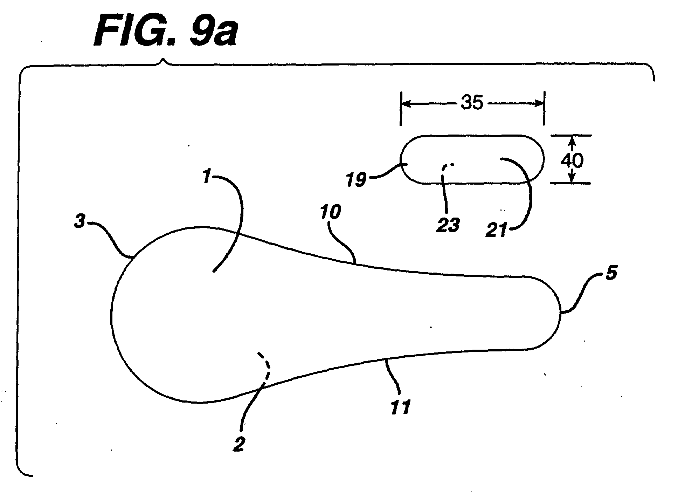

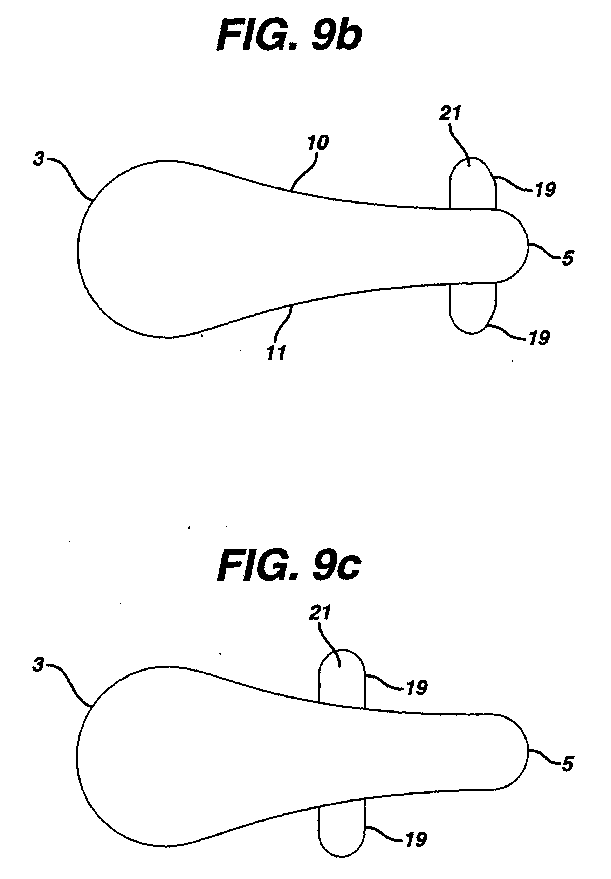

Figures 9a-c illustrate an alternate embodiment of the invention. In this embodiment, wearers wishing to have more secure attachment of an absorbent article would affix at least one attachment wing 19 to the absorbent article. As shown in Figure 9a, attachment wing 19 is a separate piece from the absorbent article. Attachment wing 19 has upper surface 21 and lower surface 23. Outer surface of backsheet 2 and lower surface 23 of attachment wing 19 may be coated with an adhesive or other separable fasteners, such as, hook and loop, snap, button, zipper, and the like. Both surfaces 2 and 23 may be protected with a release paper strip until the time of use. After release paper has been removed from outer surface of the backsheet, upper surface 21 of attachment wing 19 is affixed into any desired position on the garment-facing side of the backsheet, e.g., surface 2. For example, it may be desirable to secure the attachment wing 19 adjacent to the second portion of the absorbent article, as depicted in Figure 9b. Alternatively, the desired placement of attachment wing 19 may be in closer to first portion 3. Optionally, upper surface 21 of attachment wing 19 may have adhesive applied thereon. The use of attachment wing 19 is entirely optional and may even be omitted by the wearer.

The dimensions of the attachment wing 19 depend on where it is to be located and the type of absorbent article it is being attached to. For example, in one embodiment, the closer attachment wing 19 is located to the second portion end, the smaller length 35 will be. In a preferred embodiment, length 35 is from about 2.4 to about 2.8 inches (about 60 to about 70 mm) and width 40 is from about 0.8 to about 2.4 inches (about 20 to about 60 mm). After attachment wing 19 is secured to the absorbent article, the combined absorbent article/attachment wing combination may be secured the thong garment. While Figure 9 shows attachment wing 19 as a symmetrically formed unit, attachment wing 19 may be of any non-symmetrical shape. Additionally, it is not necessary for attachment wing 19 to have rounded edges. Attachment wing 19 may also be formed from a continuous roll similar to a roll of adhesive tape. For example, the user may dispense a strip of predetermined length from a roll where one side of the material has an adhesive coating.

The absorbent article may be applied to the crotch of a thong garment by placing the garment-facing surface against the inside surface of the crotch of the thong garment. Pressure sensitive adhesive may be applied to the garment-facing surface of the pantiliner to help maintain it in place. As used herein, the term "pressure-sensitive adhesive" refers to any releasable adhesive or releasable tenacious means. Suitable pressure sensitive adhesives include for example water-based adhesives such as acrylate adhesives. Alternatively, the adhesive may comprise "hot melt" rubber adhesives or two-sided adhesive tape.

An alternative embodiment of the present invention is a kit, wherein an absorbent article is provided as a separate piece from the wing. In the kit, the wings may be provided as individual pieces, one or more strips containing one or more wings that can be separated from each other via a perforation line, the strips may or may not be provided in roll form.

The absorbent article may include other known materials, layers, and additives, such as, foam layers, net-like layers, perfumes, medicaments, moisturizers, odor control agents, and the like, many examples of which are known in the art. The absorbent article can optionally be embossed with decorative designs using conventional techniques.

While the preferred embodiment of the invention is a pantiliner for use with a thong garment, other embodiments are also included within the scope of the present invention. For example, a removably attached wing may be used with any sanitary protection absorbent article including, but not limited to, sanitary napkin, incontinence device, and the like. Similarly, attachment wings may be added to any sanitary protection product, such as pantiliners, sanitary napkins, incontinence devices, and the like, with pantiliners being preferred, to aid in securing the absorbent article in place. Other embodiments contemplated herein include symmetrical absorbent articles having parallel longitudinal edges, dog bone- or peanut-shaped, and the like, including conventional pantiliners, sanitary napkins, incontinence devices.

From the foregoing description, one skilled in the art can ascertain the essential characteristics of this invention, and without departing from the spirit and scope thereof, can make various changes and modifications. Preferred embodiments set forth by way of illustration are not intended as limitations on the variations possible in practicing the present invention.

## Claims

1. A pantiliner comprising:
(a) a silhouette comprising
(i) a first portion having a maximum width,
(ii) a second portion having a maximum width, wherein the second portion being in opposite relation to the first portion and the maximum width of the first portion is greater than the maximum width of the second portion, and
(iii) a first longitudinally extending edge and a second longitudinally extending edge being in opposed relationship between the first portion to the second portion;
(b) a layered portion comprising
1) a cover,
2) an absorbent core, and
3) a breathable backsheet;
(c) at least one wing laterally extending from the first longitudinally extending edge or the second longitudinally extending edge; and
(d) a perforation between the wing and the longitudinally extending edge for removing the wing.

2. An pantiliner of claim 1, wherein the perforation is formed by a member selected from group consisting of knife cutting, ultrasonic cutting, embossing, and sealing.

3. An pantiliner of claim I, wherein the absorbent core comprises absorbent material selected from the group consisting of pulp fibers, bicomponent fibers, polyester fibers, and superabsorbent polymers.

4. An pantiliner of claim 1 further comprising a transfer layer.

5. An attachment wing for securing a thong pantiliner to a garment, the attachment wing comprising an upper surface, the upper surface comprising a cover and an absorbent article attachment means for securing the attachment wing to the pantiliner, and a lower surface, the lower surface comprising a garment attachment means securing the attachment wing to the garment, wherein the thong pantiliner comprises
A. a silhouette comprising
(i) a first portion having a maximum width,
(ii) a second portion having a maximum width, wherein the second portion being in opposite relation to the first portion and the maximum width of the first portion is greater than the maximum width of the second portion, and
(iii) a first longitudinally extending edge and a second longitudinally extending edge being in opposed relationship between the first portion to the second portion and
B. a layered portion comprising
(i) a cover,
(ii) an absorbent core, and
(ii) a breathable backsheet.

6. An attachment wing of claim 5, wherein the attachment wing is dispensed from a roll.

7. An attachment wing of claim 5, wherein the absorbent article further comprises a transfer layer.

8. An attachment wing of claim 5, wherein the absorbent core comprises absorbent material selected from the group consisting of pulp fibers, bicomponent fibers, polyester fibers, and superabsorbent polymers.

9. A kit for an absorbent article, the kit comprising:
A. A pantiliner comprising:
(i) a silhouette comprising
(a) a first portion having a maximum width,
(b) a second portion having a maximum width, wherein the second portion being in opposite relation to the first portion and the maximum width of the first portion is greater than the maximum width of the second portion, and
(c) a first longitudinally extending edge and a second longitudinally extending edge being in opposed relationship between the first portion to the second portion;
(ii) layered portion comprising
(a) cover,
(b) an absorbent core,
(c) a breathable backsheet; and
B. at least one attachment wing comprising a cover and a breathable backsheet.

10. A kit of claim 9, wherein the attachment wing is dispensed from a roll.

11. A kit of claim 9, wherein the absorbent core comprises absorbent material selected from the group consisting of pulp fibers, bicomponent fibers, polyester fibers, and superabsorbent polymers.

12. A kit of claim 9 further wherein the absorbent article further comprises a transfer layer.
